# EUROPÄISCHE PATENTSCHRIFT

(11) **EP 1 349 561 B1**
(45) Veröffentlichungstag und Bekanntmachung des Hinweises auf die Patenterteilung: **06.10.2004**
(21) Anmeldenummer: 02704646.5
(22) Anmeldetag: 10.01.2002
(51) Int. Cl.: A61K 35/78, A61P 31/22

(54) **ÄTHERISCHE ÖLE ZUR TOPISCHEN THERAPIE VON HERPES LABIALIS**
ESSENTIAL OILS FOR THE LOCAL TREATMENT OF HERPES LABIALIS
HUILES ESSENTIELLES POUR LE TRAITEMENT TOPIQUE DE L'HERPES LABIALIS

(30) Priorität: 10.01.2001 DE 10100809
(43) Veröffentlichungstag der Anmeldung: 08.10.2003
(73) Patentinhaber: Walther Schoenenberger Pflanzensaftwerk GmbH & Co. KG, 71106 Magstadt (DE); Heiss, Markus M., 82343 Pöcking (DE); Tarabichi, Anwar, 82205 Gilching (DE)
(72) Erfinder: HEISS, Markus, M., 82343 Pöcking (DE); TARABICHI, Anwar, 82205 Gilching (DE); GREITHER, Otto, 83052 Bruckmühl (DE)
(74) Vertreter: VOSSIUS & PARTNER
(86) Internationale Anmeldenummer: PCT/EP2002/000183
(87) Internationale Veröffentlichungsnummer: WO 2002/055056

(56) Entgegenhaltungen:
- WO-A-99/27793
- SCHNITZLER P ET AL: "Antiviral activity of Australian tea tree oil and eucalyptus oil against herpes simplex virus in cell culture." PHARMAZIE, Bd. 56, Nr. 4, April 2001 (2001-04), Seiten 343-347, XP001080122 ISSN: 0031-7144
- MARKKANEN T ET AL: "ANTI HERPETIC AGENT FROM THE JUNIPER TREE JUNIPERUS-COMMUNIS ITS PURIFICATION IDENTIFICATION AND TESTING IN PRIMARY HUMAN AMNION CELL CULTURES" DRUGS UNDER EXPERIMENTAL AND CLINICAL RESEARCH, Bd. 7, Nr. 5, 1981, Seiten 691-697, XP001080552 ISSN: 0378-6501

## Beschreibung

Diese Erfindung betrifft die topische Verwendung von ätherischen Ölen zur Therapie von Herpes labialis Typ 1.

Lokale virale Erkrankungen sind weltweit verbreitet. Vor allem Herpes labialis ist eine häufige Infektion mit den unterschiedlichsten Manifestationen, In der Bundesrepublik Deutschland sind 90% der Bevölkerung HSV-1 positiv, d.h. sie sind Träger dieses Virus, 20% dieser Träger sind symptomatisch. Diese Durchseuchungsrate ist in den Industrienationen nahezu gleich. Bei einem Anteil der Bevölkerung kommt es zu wiederholtem Auftreten der allgemein bekannten Herpesbläschen mit individuell unterschiedlicher Häufigkeit und Ausprägung. Neben den Schmerzen, die eine lokale Herpesinfektion verursacht, sind die Betroffenen zudem auf sozialer Ebene beeinträchtigt.

Die inzidenz von Organinfektionen mit dem HSV-1 Typ, wie z.B. Herpesenzephalitis oder Herpespneumonie, welche lebensbedrohlich verlaufen können und heute noch trotz des Einsatzes moderner Virustatika zu einem deutlichen Anteil letal oder mit beeinträchtigendem Residuum enden, ist von angeborenen und erworbener Formen der Immunsystemdefizite sowie von der Prävalenz der Infektion abhängig. Das bedeutet, daß die Inzidenz ernster Herpesinfektionen mit der Prävalenz der primären Infektionen direkt korreliert. Der Übertragungsweg der Infektion erfolgt hauptsächlich über Lippenkontakt im Exazerbationsstadium.

Trotz der Entwicklung diverser Virustatika (z.B. Idoxuridin, Aciclovir, Penciclovir) - als Mittel der Wahl gelten z.Zt. Penciclovir- bzw. Aciclovirhaltige Cremes mit einem Gehalt von 1% bis 5% - zeigen bisherige lokale Therapien keine zufriedenstellenden Ergebnisse in der Bekämpfung der rezidivierenden Herpesmanifestation der Lippen- und Gesichtsinfektion. Dem liegen zwei Tatsachen zugrunde. Zum einem gelangt das Virustatikum bei der topischen Anwendung nur mit niedrigen Konzentrationen ins infizierte Gewebe und zum anderen besteht die Effloreszenz auch noch nach der Elimination des Virus aus der betroffenen Schleimhaut/Haut. Sekundäre (z.B. bakterielle) Infektionen der Herpesbläschen können zudem den Verlauf komplizieren und werden von dem Virustatikum nicht beeinflußt. Außerdem werden bereits heute resistente Virusstämme gegen diese Gruppe der Chemotherapeutika entdeckt, diese Entwicklung zeigt Parallelen zur Resistenzbildung gegen Antibiotika.

Außerdem ist zu beobachten, daß virale Haut-und Schleimhautinfektionen aufgrund der Risiken virustatischer Medikamente bei der systemischen Behandlung und der insuffizienten Wirksamkeit bei der topischen Anwendung auch wegen mangelnder Patientencompliance nicht zufriedenstellend behandelt werden.

Die vorliegende Erfindung stellt ein Mittel mit höherer und schnellerer Heilungsquote bereit. Überaschenderweise wurde beobachtet, daß mit der erfindungsgemäßen topischen Applikation hochkonzentrierter, d.h. in einer Konzentration von mind. 90%, bzw. reiner Gemische aus mindestens 3 der nachfolgenden Öle: Aetheroleum Menthae piperitae, Aetheroleum Cajeputi, Aetheroleum Eucalypti, Aetheroleum Juniperi, Aetheroleum Melaleucae und Aetheroleum Gaultheriae, sich virale Haut- und Schleimhautinfektionen aufgrund eines ausreichenden Eindringens ins infizierte Gewebe mit Zerstörung der Viren und durch Förderung der Wundheilung erfolgreich behandeln lassen. Besonders bevorzugt ist hierbei die Applikation mittels eines handelsüblichen Applikationskugelrollers.

Da die akute Exazerbation der Herpesbläschen auch die Hauptinfektionsquelle für die systemische Infektion ist, kann eine erfolgreiche rasche erfindungsgemäße Behandlung des rezidivierenden Herpes labialis die Inzidenz dieser Infektion und mittel- bis langfristig epidemiologisch gesehen deren Prävalenz, welche wie oben aufgeführt mit der Inzidenz lebensbedrohender systemischer Herpesinfektionen korreliert senken.

Die antiseptische Wirksamkeit ätherischer Öle im Allgemeinen wurde mehrfach beobachtet und in experimentellen Untersuchungen bestätigt. Ätherische Öle zeigen konzentrationsabhängig mit unterschiedlichem Ausprägungsspektrum der Einzelsubstanzen keimhemmende Wirkungen gegen einzelne Bakterien (4,6,7,9,10,12), Pilze (8,14) und Viren (15,16). So zeigen Laurylaldehyd, Caprylaldehyd, iso-Borneol, Borneol und 1,8-Cineol in in-vitro Untersuchungen antivirale Eigenschaften wenn sie in einem Konzentrationsbereich von 0.03% bis 1% eingesetzt werden, wobei der zu Grunde liegende Mechanismus im Einzelnen noch nicht bekannt ist und eine Verallgemeinerung auf ätherische Öle und ihre Bestandteile nicht möglich ist. So inhibiert iso-Borneol die virale Glycosylierung während sein Stereoisomer Borneol und auch 1,8-Cineol die Glycosylierung nicht inhibieren. Zudem zeigen bestimmte ätherische Öle eine Steigerung der Durchblutung bei topischer Anwendung (5) und eine analgetische Potenz (2, 13).

Pharmakologisch gesehen können emsthafte Nebenwirkungen durch Überdosierungen beim normalen therapeutischen Einsatz bei Weitem nicht erreicht werden. Zudem gelten ätherische Öle toxikologisch gesehen als für den Menschen gut verträglich. Vergiftungen durch übermäßige Ingestionen sind äußerst selten, da dies durch die Geruchs- und Geschmacksintensität der Öle verhindert wird. Auch in Destillatform wurden bei üblicher Dosierung noch keine ernsthaften Nebenwirkungen, bzw. frühe oder späte Komplikationen beobachtet. Wissenschaftlich fundierte Daten zur Toxikologie, Pharmakokinetik und Pharmakodynamik diverser ätherischer Öle (1,3) liegen vor.

Zwar ist der Einsatz der Ätherischöl-Drogen v.a. in der Volksmedizin weltweit verbreitet, ihre therapeutischen Effekte werden bisher in Form von niedrig konzentrierten Präparaten in Aufgüssen, Extrakten, Tinkturen und in Cremen und Salben genutzt. Bislang wurde jedoch die topische Anwendung von hochkonzentrierten oder unverdünnten ätherischen Ölen auf geschädigter Haut gemeinhin wegen der allgemein bekannten hautreizenden Wirkung auf intakter Haut nicht in Erwägung gezogen bzw. galt sogar als kontrainduziert.

Ein ethanolischer Auszug von *Salvia officinalis ssp. Minor* GAMS ist ein Bestandteil einer bei Infektionen der Mund- und Rachenschleimhäute verwendeten Zubereitung (Viru-Salvysat® Viskose Lösung). Zudem besitzt eine wässrige Extraktfraktion von *Melissa offcinalis* (Lomaherpan®) eine antivirale Wirkung die vermutlich auf einer Bindung der "Lamiaceengerbstoffe" an Virus- und Zellmembranproteine beruht, wodurch eine Adsorption der Viren an die Zellmembran verhindert wird.

In der Schweizer Patentschrift CH 688 787 A5 werden kursorisch Zusammensetzungen aus verschiedensten ätherischen Ölen mit antiseptischer, antimikrobieller, antiviraler, antibiotischer bakterizider, bakteriostatischer, desinfizierender, fungizider, fungistatischer, antiparasitärer, entzündungshemmender, hautpflegender, juckreizmildernder, heilungs-, wachstums-, und/oder durchblutungsfördernder Wirkung beschrieben. Jedoch handelt es sich hierbei im Gegensatz zu den erfindungsgemäßen Zusammensetzungen nicht um hochprozentige Zusammensetzungen zur Applikation auf offenen Wunden, sondern um niedrig konzentrierte Zusammensetzungen (1 bis 10%), etwa zur Verwendung als Repellent für Haustiere, als ungezieferabtötendes Shampoo für Haustiee, als Pfoten- oder Fellpflegemittel, sowie als Pflanzenschutzmittel und Insektenrepellent.

Die vorliegende Erfindung stellt nun ein hochprozentiges Mittel von mindestens 90% zur topischen Behandlung von viralen Haut- und Schleimhauterkankungen zur Verfügung, enthaltend mindestens drei der nachfolgend genannten Öle: Aetheroleum Menthae piperitae, Aetheroleum Cajeputi, Aetheroleum Eucalypti, Aetheroleum Juniperi, Aetheroleum Melaleucae und Aetheroleum Gaultheriae.
Weiterhin zu betonen ist hierbei die zusätzlich zu beobachtende schmerzstillende, lokalanästhetische Wirkung dieser erfindungsgemäßen Gemische, welche natürlich bei der Behandlung von viralen Effloreszenzen besonders vorteilhaft ist.

Besonders bevorzugt sind Mischungen aus den folgenden fünf der oben genannten ätherischen Öle Aetheroleum Menthae piperitae, Aetheroleum Cajeputi, Aetheroleum Eucalypti, Aetheroleum Juniperi, und Aetheroleum Gaultheriae. Femer werden Mischungen wobei Aetheroleum Menthae piperitae, Aetheroleum Cajeputi und Aetheroleum Eucalypti die Hauptwirkstoffe bzw. die einzigen Wirkstoffe sind bevorzugt.
Eine ganz besonders bevorzugte Ausführungsform der Erfindung weist die folgende Zusammensetzung auf:

| | |
|---|---|
| Aetheroleum Menthae piperitae | 53% |
| Aetheroleum Cajeputi | 21% |
| Aetheroleum Eucalypti | 21% |
| Aetheroleum Juniperi | 3% |
| Aetheroleum Gaultheriae | 2%. |

Als ätherische Öle welche für die erfindungsgemäßen Gemische verwendet werden können sind sowohl die natürlichen ätherischen Öle, als auch synthetische (naturidentische) Produkte - bestehend aus einem oder der Kombination mehrerer der Hauptinhaitstoffe des entsprechenden natürlichen Öls - zu verstehen. Besonders bevorzugt ist hierbei die Verwendung der entsprechenden natürlichen ätherischen Öle.

Die in den erfindungsgemäßen Gemischen zu verwendenden natürlichen ätherischen Öle werden durch die dem Fachmann bekannten Destillationsverfahren gewonnen, wobei im einzelnen die Wasser-Destillation, die Wasser-/Dampfdestillation und die Dampfdestillation zu nennen sind. Weitere verwendete Gewinnungsverfahren sind das Enfleurage-Verfahren (Ölextraktion), die Extraktion mit organischen Lösungsmitteln, sowie die Kaltpressung (Auspreßverfahren). Die so erhaltenen Öle, v.a. die wasserdampfdestillierten Öle können anschließend durch fraktionierte Destillation über Kolonnen (Rektifikation) weiter aufgereinigt werden.

Besonders bevorzugt ist die erfindungsgemäße topische Applikation von 100% tigen Gemischen aus mindestens 3 der nachfolgenden Öle: Aetheroleum Menthae piperitae, Aetheroleum Cajeputi, Aetheroleum Eucalypti, Aetheroleum Juniperi, Aetheroleum Melaleucae und Aetheroleum Gaultheriae ohne Zusätze, in der Behandlung von Haut- und Schleimhauteffloreszenzen viraler Infektionen, im besonderen von Herpes labialis Typ 1, sowie eine hochprozentige Anwendung. Wie bereits oben erwähnt wird hierunter eine Konzentration von mindestens 90%verstanden.

Eine weitere bevorzugte Ausführungsform der Erfindung ermöglicht die erfindungsgemäße topische Applikation von hoch konzentrierten, d.h. mindesten 90% tigen hydrophilen oder lipophilen Zubereitungen.

Hierbei wird eine neue optimale Therapieform, die viele Vorteile aufweist und bis heute auch nicht mit den modernen Virusstatika erreichte Behandlungsergebnisse zeigt, bereitgestellt. Die lokal einsetzbare Medikation besitzt eine hohe Permeabilität in das Gewebe (11) durch welche eine therapeutisch einsetzbare viruzide Konzentration im infizierten Gewebe erreicht wird. Eine schädigende Nebenwirkung ist bei bestimmungsgemäßer Anwendung ausgeschlossen, da - wie bereits oben ausgeführt - toxische Komplikationen erst bei Konzentrationen auftreten können, die bei topischer Applikation nicht erreicht werden können. Die ursächliche Behandlung durch die Bekämpfung der viralen Infektion und die direkte anaigetische Wirksamkeit der ätherischen Öle (2,13) bedingen eine rasche Schmerzlinderung oder sogar - beseitigung. Da ätherische Öle auch gegen sonstige Mikroorganismen wirksam sind, wird zudem eine bakteriell oder mykos bedingte Sekundärinfektion verhindert bzw. ebenfalls behandelt. Die hyperämisierende Wirkung ätherischer Öle (5), die bei hohen Konzentrationen bzw. bei dem Einsatz von Destillaten ätherischer Öle nachgewiesen wurde, beschleunigt zudem die Regeneration und somit die Abheilung.

Durch Eindringen höherer Konzentrationen dieser lipophilen erfindungsgemäßen Gemische aus mindestens 3 der nachfolgenden Öle: Aetheroleum Menthae piperitae, Aetheroleum Cajeputi, Aetheroleum Eucalypti, Aetheroleum Juniperi, Aetheroleum Melaleucae und Aetheroleum Gaultheriae mit entsprechend höher Permeabilität ins infizierte Gewebe werden die viralen Primärerreger in tieferen Gewebeschichten eliminiert. Die Steigerung der lokalen Durchblutung im Wundgebiet und im umgebenden Gewebe führt zur Beschleunigung regeneratorischer Mechanismen. Auch die Funktionen des Immunsystems (Immigration der Makrophagen, Übertritt von Antikörpern) kommen besser zum Tragen. Es wurde zudem beobachtet, daß ätherische Öle trotz Steigerung der Durchblutung gleichzeitig eine Hemmung entzündlicher Reaktionen bewirken, was zur Herstellung des physiologischen Gleichgewichts führt. Eine potentielle sekundäre Suprainfektion wird ebenfalls behandelt. Die Analgesie ist ein Nebeneffekt, der gerade in der akuten Phase bedeutsam ist. Hierbei ist nochmals zu betonen, dass bisher ätherische Öle als auf die verletzte Haut und Schleimhaut als schmerzauslösend gehalten wurden, da sie eine typische (wenn auch in der Regel angenehme) Reizung auf die intakte Haut verursachen. Eine über die Keimschädigung hinausgehende, die schnellere Abheilung fördernde Wirkung war nicht zu erwarten.

Diese Entdeckung wurde im Rahmen einer klinischen Studie untersucht und bestätigt.

### In vivo Studie

Die Wirksamkeit der erfindungsgemäßen Verwendung des unten in Beispiel1 beschriebenen Zusammensetzung wurde an 30 Probanden mit labialer Rezidivmanifestation von HSV-1-Infektion untersucht. Die Zeitspanne bis zur völligen Abheilung mit gänzlichem Verschwinden der Herpesbläschen (Effloreszenz) lag bei 28 Probanden unabhängig vom Entwicklungsstadium der Effloreszenz bei 2 bis 6 Tagen (Median 3,6 Tage). Die Schmerzen waren bereits am ersten Tag bei allen Probanden abgeklungen. Im Rahmen der Abklärung der optimalen Dosis wurde die Applikation von einmal 2-bis 3-stündlich auf zweimal stündlich bei zwei Probanden durchgeführt. Bei diesen zwei Probanden mit der häufigeren Applikation (zweimal stündlich- bzw. ca. 20 mal täglich) kam es zu einer optischen und klinischen Befundverschlechterung, die letztendlich eine Überdosierung anzeigte. Diese zwei ungünstigen Verläufe mit dem Abbrechen der Behandlung nach zwei Tagen sind mit der übermäßigen hautentfettenden Wirkung der ätherischen Öle zu begründen. Weitere Nebenwirkungen sind bei diesen zwei Therapieversagem nicht aufgetreten.

Diese Behandlungsergebnisse übertreffen die aktuellsten Behandlungsergebnisse mit dem neuesten bis heute am effektivsten topisch angewendeten Virustatikum Penciclovir (Benchmark Virustatikum) und stellt somit überlegene Arzneimittel zur Behandlung von viralen Hauterkrankungen, insbesondere von Herpes labialis Typ1, zur Verfügung.

Zudem läßt die Nachbeobachtung der Probanden auf eine deutliche Senkung der Rezidivquote schliessen.

### Beispiele

### Beispiel 1:

Folgendes Mischpräparat ätherischer Öle wurde auf die befallene Haut bzw. Halbschleimhaut der Lippen appliziert. Die Häufigkeit der Anwendung sollte erfindungsgemäß zwischen 4 bis 8 mal täglich liegen.

| | |
|---|---|
| Aetheroleum Menthae piperitae | 53% |
| Aetheroleum Cajeputi | 21% |
| Aetheroleum Eucalypti | 21% |
| Aetheroleum Juniperi | 3% |
| Aetheroleum Gaultheriae | 2% |

Die aufgetragene Menge sollte so bemessen sein, daß ein deutlich sichtbarer Flüssigkeitsfilm appliziert wird.

### Beispiel 2

Ein Mischpräparat wie in den vorstehenden Beispiel beschrieben wird mit Glycerin, Vaseline oder einer anderen dem Fachmann bekannten lipophilen hautpflegenden Substanz verdünnt.

Nach Solubilisierung mit Lösungsvermittlem wie z.B. Tween 80®, können die erfindungsgemäß zu verwendenden ätherischen Öle auch in hydrophilen Trägern formuliert werden.

Selbstverständlich können die erfindungsgemäß zu verwendenden Gemische auch in anderen als den hier aufgeführten dem Fachmann als geeignet bekannten Vehikeln oder Grundlagen formuliert werden.

Besonders bevorzugt ist zudem die erfindungsgemäße Verwendung mittels Applikation mit einem handelsüblichen Applikationkugelroller.

### Literatur

- 1.: Delgado IF, Carvalho RR, Nogueira AC, Mattos AP, Figueiredo LH, Oliveira SH, Chahoud I,
Paumgartten FJ, Study on embryo-foetotoxicity of beta-myrcene in the rat. Food-Chem-Toxicol, Jan 1993 31 (1) 31-5
- 2.: Göbel H, Schmidt G, Soyka D
Effect of peppermint and eucalyptus oil preparations on neurophysiological and experimental algesimetric headache parameters.
Cephalalgia, Jun 1994 14(3) 228-34
- 3.: Guseinov D, Kagramanov KM, Kasumov F, Akhundov RA
Research on the chemical composition and aspects of the pharmacological action of the essential oil of Kochi thyme (Thymus kotschyanus Boiss). Farmakol-Toxikol, Mar-Apr 1987 50(2) 73-74
- 4.: Harkenthal M, Reichling J, Geiss HK, Saller R
Comperative study on the in vitro antibacterial activity of Australien tee tree oil, cajeput oil, manuka oil, and eucalyptus oil.
Pharmazie, 1999 54(6) 460-63
- 5.: Hong CZ, Shellock FG
Effects of topically applied counterirritant (Eucalyptamint) on cutaneous blood flow and on skin and muscle temperatures. A placebo-controlled study.
Am J Phys Med Rehabil, Feb 1991 70(1) 29-33
- 6.: Jedlickova Z, Mottl O, Sery V
Antibacterial properties of the vietnamese cajeput oil and oclmum oil in combination with antibactrial agents.
J Hyg Epidemiol Microbiol Immunol (Czechoslovakia), 1992 36(3) 303-9
- 7.: Kalodera Z, Pepeljnjak S, Vladimir S, Blazevic N
Antimicrobial activity of essential oil from Micromeria thymifolia.
Pharmazie, May 1994 49(5) 376-7
- 8.: Kishore N, Mishra AK, Chansouria JP
Fungitoxicity of essential oils against dermatophytes.
Mycoses, May-Jun 1993 36(5-6) 211-5
- 9.: Marsh PD
Microbiological aspects of the chemical control of plaque and gingivitis J Dent Res, Jul 1992 71(7) 1431-8
- 10.: Moleyar V, Naraslmham P
Antibacterial activity of essential oil components
Int J Food Microbiol, Aug 199216(4) 337-42
- 11.: Obata Y, Takayama K, Okabe H, Nagai T
Effect of cyclic monotetpenes on percutaneous absorption in the case of a water-soluble drug (diclofenac sodium).
Drug Des Deliv Oct 1990 6(4) 319-28
- 12.: Panizzi L, Flamini G, Cioni PL, Morelli I
Composition and antimicrobial properties of essential oils of four Mediterranean Lamiaceae.
J Ethnopharmacol ,Aug 1993 39(3) 167-70
- 13.: Siemleniuk A, Szalkowska-Pagowska H, Lochynski S, Piatkowskl K, Filipek B Krupinska J, Czarnecki R, Librowski T, Zebala K
Synthesis and local anesthetic and circulatory actions of aminoesters and hydroxyamine monoterpene derivatives.
Pol J Pharmacol Pharm, Jul-Aug 1992 44(4) 407-20
- 14.: Tong MM, Altman PM, Bametson R S
Tea tree oil in the treatment on tinea pedis
Australas-J-Dermatol, 1992, 33 (3) 145-9
- 15.: Armaka M, Papanikolaou E, Sivropoulou A; Arsenakis M
Antiviral properties of isobomeol, a potent inhibitor of herpes virus simplex type 1.
Antiviral Res, 1999 Sep 43(2) 79-92
- 16.: Hayashi K, Kamiya M, Hayashi T
Virucidal effects of the steam distillate from Houttuynia cordata and its components on HSV-1, influenza virus and HIV.
Planta Med ,1995 Jun 61 (3) 237-41

## Patentansprüche

1. Verwendung eines Gemisches aus mindestens 3 der nachfolgenden Öle:
Aetheroleum Menthae piperitae, Aetheroleum Cajeputi, Aetheroleum Eucalypti, Aetheroleum Juniperi, Aetheroleum Melaleucae und Aetheroleum Gaultheriae, zur Herstellung eines Arzneimittels zur topischen Applikation zur Therapie von Herpes labialis Typ 1 Infektionen der Haut oder der Schleimhäute in einer Konzentration von mindestens 90%.

2. Verwendung nach Anspruch 1, wobei das Gemisch unverdünnt anzuwenden ist.

3. Verwendung nach Anspruch 1, wobei das Gemisch in Form von wässrigen oder öligen Lösungen vorliegt.

4. Verwendung nach Anspruch 3, wobei das Gemisch in Glycerin, Vaseline oder sonstiger hautpflegender Substanz verdünnt wird.

5. Verwendung nach Anspruch 3, wobei das Gemisch in Glycerin mit Hilfe eines geeigneten Lösungsmittels wasserlöslich gemacht wird und in hydrophiler Lösung verdünnt wird.

6. Verwendung nach einem der Ansprüche 1-5, wobei das Gemisch Aetheroleum Menthae piperitae, Aetheroleum Cajeputi und Aetheroleum Eucatypti enthält.

7. Verwendung nach einem der Ansprüche 1-5, wobei das Gemisch Aetheroleum Menthae piperitae, Aetheroleum Cajeputi, Aetheroleum Eucalypti und Aetheroleum Juniperi enthält.

8. Verwendung nach einem der Ansprüche 1-5, wobei das Gemisch Aetheroleum Menthae piperitae, Aetheroleum Cajeputi, Aetheroleum Eucalypti, Aetheroleum Juniperi und Aetheroleum Gaultheriae enthält.

9. Verwendung nach einem der Ansprüche 1-6, wobei das Gemisch die folgende Zusammensetzung enthält:
| | |
|---|---|
| Aetheroleum Menthae piperitae | 53% |
| Aetheroleum Cajeputi | 21% |
| Aetheroleum Eucalypti | 21% |

10. Verwendung nach einem der Ansprüche 1 bis 9, wobei das Gemisch die folgende Zusammensetzung aufweist:
| | |
|---|---|
| Aetheroleum Menthae piperitae | 53% |
| Aetheroleum Cajeputi | 21% |
| Aetheroleum Eucalypti | 21% |
| Aetheroleum Juniperi | 3% |
| Aetheroleum Gaultheriae | 2%. |

11. Verwendung nach einem der Ansprüche 1-10, wobei die Applikation mittels eines Applikationskugelrollers erfolgt.

## Claims

1. Use of a mixture of at least three of the following oils: Menthae piperitae aetheroleum, Caeputi aetheroleum, Eucalypti aethereoleum, Juniperi aetheroleum, Melaleucae aetheroleum and Gaultheriae aetheroleum in a concentration of at least 90% for the preparation of a pharmaceutical composition for topical administration in treating Herpes labialis type I infections of the skin or mucosa.

2. Use according to claim 1 wherein the mixture is to be used undiluted.

3. Use according to claim 1 wherein the mixture is present in the form of aqueous or oily solutions.

4. Use according to claim 3 wherein the mixture is diluted in glycerol, paraffin jelly or another skin-care substance.

5. Use according to claim 3 wherein the mixture in glycerol is rendered water-soluble by means of a suitable solubiliser and is diluted in a hydrophilic solution.

6. Use according to any one of claims 1 to 5 wherein the mixture contains Menthae piperitae aetheroleum, Caeputi aetheroleum and Eucalypti aetheroleum.

7. Use according to any one of claims 1 to 5 wherein the mixture contains Menthae piperitae aetheroleum, Caeputi aetheroleum, Eucalypti aetheroleum and Juniperi aetheroleum.

8. Use according to any one of claims 1 to 5 wherein the mixture contains Menthae piperitae aetheroleum, Caeputi aetheroleum, Eucalypti aethereoleum, Juniperi aetheroleum and Gaultheriae aetheroleum.

9. Use of any one of claims 1 to 6 wherein the mixture contains the following composition:
| | |
|---|---|
| Menthae piperitae aetheroleum | 53% |
| Caeputi aetheroleum | 21% |
| Eucalypti aetheroleum | 21 %. |

10. Use of any one of claims 1 to 9 wherein the mixture has the following composition:
| | |
|---|---|
| Menthae piperitae aetheroleum | 53% |
| Caeputi aetheroleum | 21% |
| Eucalypti aetheroleum | 21% |
| Juniperi aetheroleum | 3% |
| Gaultheriae aetheroleum | 2%. |

11. The use of any one of claims 1 to 10, wherein the application is carried out with a roll-on device for application.

## Revendications

1. Utilisation d'un mélange d'au moins 3 des huiles suivantes : Aetheroleum Menthae piperitae, Aetheroleum Cajeputi, Aetheroleum Eucalypti, Aetheroleum Juniperi, Aetheroleum Melaleucae et Aetheroleum Gaultheriae , pour la fabrication d'un médicament pour application topique pour la thérapie d'infections de la peau ou des muqueuses dues à Herpes Lablalis de type 1, en une concentration d'au moins 90 %.

2. Utilisation selon la revendication 1, dans laquelle le mélange est à utiliser sans dilution.

3. Utilisation selon la revendication 1, dans laquelle le mélange se présente sous la forme des solutions aqueuse ou huileuse.

4. Utilisation selon la revendication 3, dans laquelle le mélange est dilué dans de la glycérine, de la vaseline ou une autre substance destinée au soin de la peau.

5. Utilisation selon la revendication 3, dans laquelle le mélange dans la glycérine est rendu soluble dans l'eau à l'aide d'un agent de solubilisation approprié et dilué dans une solution hydrophile.

6. Utilisation selon l'une quelconque des revendications 1 à 5, dans laquelle le mélange contient Aetheroleum Menthae piperitae, Aetheroleum Cajeputi et Aetheroleum Eucalypti.

7. Utilisation selon l'une quelconque des revendications 1 à 5, dans laquelle le mélange contient Aetheroleum Menthae piperitae, Aetheroleum Cajeputi, Aetheroleum Eucalypti et Aetheroleum Juniperi.

8. Utilisation selon l'une quelconque des revendications 1 à 5, dans laquelle le mélange contient Aetheroleum Menthae piperitae, Aetheroleum Cajeputi, Aetheroleum Eucalypti, Aetheroleum Juniperi et Aetheroleum Gaultheriae.

9. Utilisation selon l'une quelconque des revendications 1 à 6, dans laquelle le mélange comprend la composition suivante :
| | |
|---|---|
| Aetheroleum Menthae piperitae | 53 % |
| Aetheroleum Cajeputi | 21 % |
| Aetheroleum Eucalypti | 21 % |

10. Utilisation selon l'une quelconque des revendications 1 à 9, dans laquelle le mélange présente la composition suivante :
| | |
|---|---|
| Aetheroleum Menthae piperitae | 53 % |
| Aetheroleum Cajeputi | 21 % |
| Aetheroleum Eucalypti | 21 % |
| Aetheroleum Juniperi | 3 % |
| Aetheroleum Gaultheriae | 2 %. |

11. Utilisation selon l'une quelconque des revendications 1 à 10, dans laquelle l'application a lieu au moyen d'un applicateur à bille.
